Europäisches Patentamt

European Patent Office

Office européen des brevets

(11) Publication number: 0 408 370 B1
# EUROPEAN PATENT SPECIFICATION

(45) Date of publication of patent specification: **13.09.95**  (51) Int. Cl.⁶: **A61K 9/10**, A61K 31/07

(21) Application number: **90307656.0**

(22) Date of filing: **12.07.90**

The file contains technical information submitted after the application was filed and not included in this specification

(54) **Stable tretinoin emulsified cream formulations.**

(30) Priority: **13.07.89 US 379294**
**20.12.89 US 453640**

(43) Date of publication of application:
**16.01.91 Bulletin 91/03**

(45) Publication of the grant of the patent:
**13.09.95 Bulletin 95/37**

(84) Designated Contracting States:
**AT BE CH DE DK ES FR GB GR IT LI LU NL SE**

(56) References cited:
EP-A- 0 350 438      GB-A- 906 000
GB-A- 1 466 062      US-A- 3 906 108
US-A- 3 966 967      US-A- 4 727 088

**USAN and the USP Dictionary of Drug Names,p.613**

(73) Proprietor: **Bristol-Myers Squibb Company**
**345 Park Avenue**
**New York, N.Y. 10154 (US)**

(72) Inventor: **Parab, Prakash**
**267 Patton Place**
**Williamsville,**
**New York 14221 (US)**

(74) Representative: **Jones, Alan John et al**
**CARPMAELS & RANSFORD**
**43 Bloomsbury Square**
**London, WC1A 2RA (GB)**

**Description**

Background of the Invention

This invention relates to stable tretinoin (also known as all-trans retinoic acid and vitamin A acid) cream formulations.

Acne vulgaris, the most common skin disease in the United States, affects some 17 million people. It is found most frequently in those aged 12 to 25 years old and accounts for about one-fifth of the practice of dermatology.

The acne lesions occur in sebaceous follicles, with maximum density existing on the face, chest and back. The characteristic lesion of acne is a comedone. Blackheads are open comedones, so colored due to melanin deposition. The follicle is filled with keratin, lipids and bacteria, and has a widely dilated orifice that allows its contents to escape.

Closed comedones, or whiteheads, are small and flesh colored and have microscopic openings that prevent the contents from escaping. Continued production of keratin and sebum may, however, lead to the rupture of the whitehead, releasing the contents into the dermis and subsequently initiating an inflammatory process. Inflammation close to the skin's surface results in the formation of papules and pustules, whereas in deeper sites the result is the formation of nodules and cysts. In severe cases, acne vulgaris will lead to permanent scarring, causing the patient mental pain and suffering.

A. M. Kligman (U.S. Patent 3,729,568) discloses the use of Vitamin A acid in the treatment of acne. This therapy was found to be markedly effective compared to prior methods, such as the use of peeling agents, hormone therapy (in females), antibacterial treatment and surgical skin planing.

More recently, A. M. Kligman (U.S. Patent 4,603,146) discloses the use of tretinoin to retard the aging of human facial skin. Normal aging and exposure to environmental traumas and sunlight lead to decreases in cellular turnover rates in the epidermis, a decrease in collagen fibers and the formation of abnormal elastic fibers in the dermis, causing the loss of elasticity and wrinkling of the skin. A leather-like consistency, mottling (hyperpigmentation), and various premalignant growths are also associated with the photoaging of the skin.

The elderly tend to have thin skin with decreased blood flow, accompanied by the skin's loss of sensory acuity and ability to heal. Tretinoin increases the proliferation of epidermal cells, thus thickening the epidermis, correcting atrophy and facilitating healing. Tretinoin also corrects the abnormal cell differentiation found in sun-damaged skin and reduces or stops hyperpigmentation. Furthermore, tretinoin stimulates dermal fibroblasts to produce new collagen and ground substance such as mucopolysaccharides, resulting in livelier, smoother, tighter skin possessing increased turgor. Finally, tretinoin stimulates blood flow and helps in the formation of new blood vessels in the skin. This in turn increases the following: skin temperature, clearance of irritants and toxins from the skin and sensitivity to pain and irritation.

In addition to acne treatment and treatment for photoaging, natural aging and other environmental traumas, other uses of Vitamin A acid have been reviewed by Thomas and Doyle (The Journal of the American Academy of Dermatology, Vol. 4, No. 5; May, 1981). These uses include the treatment of ichthyosis, psoriasis, acanthosis nigricans, lichen planus, molluscum contagiosum, reactive perforating collagenosis, melasma, geographical tongue, corneal epithelial peeling, Fox-Fordyce disease, cutaneous metastatic melanoma, keloids, hypertrophic scars, linear verrucous nevus, plantar warts, pseudofolliculitis, keratoacanthoma, solar keratosis of extremities, callosities and Darier's disease.

In the treatment of acne, A. M. Kligman (U.S. Patent 3,729,568) discloses solution formulations of tretinoin. The drug was dissolved in water miscible organic solvents such as ethyl alcohol, propylene glycol, polyethylene glycol and isopropyl alcohol. U.S. Patent 4,247,547 to Marks discloses a tretinoin gel formulation containing organic solvents, such as ethyl alcohol, isopropyl alcohol and propylene glycol thickened with hydroxypropyl cellulose. This gel composition was demonstrated to be highly effective in treating acne and is capable of being stored without refrigeration as well.

The prior art has used organic solvents that are potential irritants and or stinging agents. Although propylene glycol has been used in topical formulations for quite some time, it has a history of causing irritation and sensitization (C. Huriez, "Allergy to Propylene Glycol", Rev. Franc. Allerg. 6:200, 1966; M. Hannuksela, V. Pivila and O.P. Salo, "Skin Reactions to Propylene Glycol", Contact Dermatitis, 1:112, 1975; S. Aagren-Jonsson and B. Magnusson, "Sensitization to Propantheline Bromide, Trichlorocarbanilide and Propylene Glycol in Anti-Perspirants", Contact Dermatitis, 2:79, 1976).

Furthermore, the glycols, for example propylene glycol and PEG-400, are hygroscopic. When used in higher concentrations, they may absorb water from the skin, thus making it dry and irritated. The alcohols, such as ethyl alcohol and isopropyl alcohol, are also well known for causing stinging and drying effects when applied topically.

Tretinoin is known to be an irritant when applied topically (A. M. Kligman, U.S. Patent 3,729,568). Hence, it would be desirable to formulate tretinoin products devoid of stinging, irritating and sensitizing agents. One solution is a cream formulation in which either oil is dispersed in water or water is dispersed in oil. Emollients or lubricating agents present in the cream will be beneficial because they may reduce the erythema, itching and stinging associated with tretinoin. Also, water will hydrate the skin, reducing the irritation caused by dry skin.

A cosmetic cream formulation having Vitamin A acid for the regulation of cornification in human skin is disclosed in British Patent 906,000. No mention is made of its use for the treatment of acne.

British Patent 1,466,062 discloses both cream and solution formulations of tretinoin that bring marked cosmetic improvement to the skin and also condition the nails and hair.

Retin-A, a cream formulation containing tretinoin, has been produced for the commercial market. This formulation is disclosed in Physicians' Desk Reference, 43d Edition, page 1517 (1989). The formulation is described as containing tretinoin in either of two strengths, 0.1% or 0.05% by weight, in a hydrophilic cream vehicle of stearic acid, isopropyl myristate, stearyl alcohol, xanthan gum, sorbic acid, butylated hydroxytoluene, and purified water.

U.S. Patent 3,906,108 to Felty discloses a stabilized cream emulsion of tretinoin, capable of being stored without refrigeration for long periods of time without losing therapeutic effectiveness and while maintaining the uniformity and stability of the cream, containing xanthan gum as the stabilizer to prevent breaking of the emulsion. A "Declaration Pursuant to Rule 132" filed during the prosecution of U.S. Patent 3,906,108 discloses an unstable, comparative formula containing no xanthan gum, and 3.0% by weight of stearyl alcohol.

The United States Pharmacopeia (USP) XXI, page 1075, specifies that during its shelf life, the marketed tretinoin cream should contain not less than 90% and not more than 130% of the labelled amount of tretinoin. The 30% overage allowed by the USP is due to the instability of tretinoin in cream formulations. Tretinoin may degrade by free radical mediated oxidation, photochemical reactions, heat mediated reactions, and isomerization. Furthermore, tretinoin may react with the excipients in the formulation, resulting in degraded by-products. These by-products may be irritating, sensitizing or phototoxic, or may have unknown side effects. Hence, it would be preferable to formulate a tretinoin cream product for the market in which degradation is minimal. This would result in a reduction of by-products, and therefore side effects, during the product shelf-life.

U.S. Patent 4,727,088 to Scott et al discloses an anhydrous pharmaceutical preparation for the treatment of acne containing tretinoin, volatile silicones and a mixture of stearyl and cetyl alcohol.

U.S. Patent 4,837,213 to Caron et al discloses a stable anhydrous gel of tretinoin containing paraffin oil, fatty acid ester and silicone. The patent states that the anhydrous composition is useful as a carrier for oxidation sensitive dermatological medicaments such as cis- or trans-retinoic acid and anthralin.

EP-A-0350438 which was published on 10th January 1990 discloses a composition for cosmetic or pharmaceutical use containing as active ingredient an acid or ester of vitamin A in combination with a stable aqueous emulsion of hydrogen peroxide.

US-A-3966967 concerns the promotion of wound healing, in the treatment of acne, psoriasis and related skin disorders by applying to the site as a solution, ointment or powder, the $C_{20}$ and $C_{22}$ vinylogs of desmethyl retinoic acid.

Summary of the Invention

Emulsified aqueous cream formulations of tretinoin, which do not contain xanthan gum and which are capable of being stored without refrigeration for long periods of time without losing therapeutic effectiveness and while maintaining physical and chemical stability, have been discovered. Physical stability refers to the stability of the emulsion, i.e., whether or not it breaks upon storage, whereas chemical stability refers to the amount of tretinoin remaining in the emulsion after storage. According to this invention from 4 to 30% by weight of stearyl alcohol, or 12 to 30% by weight of cetyl alcohol, or cetyl alcohol and 4 to 30% by weight of stearyl alcohol is used to stabilise an emulsified aqueous tretinoin cream formulation comprising a therapeutically active amount of tretinoin and a pharmaceutically acceptable aqueous carrier whereby breaking of the emulsion is prevented

Detailed Description of the Invention

In a preferred embodiment, the emulsified aqueous cream formulation of this invention contains from about 0.005% to about 0.5% by weight of tretinoin; from about 1 to about 15% by weight of primary emulsifier; from about 10 to about 50% by weight of a hydrophobic material such as fatty acids having 12 to 20 carbon atoms, fatty acid esters in which the fatty acids have 6 to 20 carbon atoms, fatty alcohols having 12 to 20 carbon atoms, cocoa butter, mineral oil, petrolatum, beeswax, white wax, hydrocarbon wax, sperm wax, and silicone oils; from about 0.05 to about 5% by weight of preservatives; from about 0.01 to about 1% of antioxidants; from about 4 to about 30% by weight of stearyl alcohol alone or in combination with cetyl alcohol or 12 to 30% by weight of cetyl alcohol alone as a stabilizer to prevent breaking of the emulsion; and water.

The amount of stearyl alcohol or cetyl alcohol needed will depend upon the percentage of oil phase in the emulsion. The higher the oil content, the higher the amount of stearyl alcohol or cetyl alcohol required and vice-versa. Thus, the amount of stearyl and cetyl alcohol required has to be optimized, depending on the percentage of oil phase present. It is also preferred to use a blend of emulsifiers with high and low HLB values.

When a combination of cetyl alcohol and 4 to 30% by weight of stearyl alcohol is used, it is preferred to use from 3 to 6% by weight of cetyl alcohol. This combination has been found to be synergistic.

In a more preferred embodiment, the formulation contains from about 4 to about 10% by weight of stearyl alcohol, especially 6 to 9% by weight of stearyl alcohol or 4-30% by weight of stearyl alcohol and at least 3% by weight of cetyl alcohol. The primary emulsifier may be either a non-ionic emulsifier, a cationic emulsifier, an anionic emulsifier, or a mixture thereof. Examples of suitable non-ionic emulsifiers include polyoxyethylene-2-stearyl ether, polyoxyethylene-21-stearyl ether, polyoxyethylene-4-lauryl ether, polyethylene glycol-23-lauryl ether, sorbitan monostearate and polyoxyethylene-20-sorbitan monostearate. An example of a suitable anionic emulsifier is sodium lauryl sulfate and a suitable cationic emulsifier is cetyl trimethyl ammonium bromide. Suitable preservatives include diazolidinyl urea, benzyl alcohol, methyl paraben, propyl paraben, DMDM hydantoin (1,3-dimethylol-5,5-dimethyl hydantoin), iodo propynyl butyl carbamate, methylisothiazolinone and methylchloroisothiazolinone. Suitable antioxidants include butylated hydroxyanisole, butylated hydroxytoluene, tocopherol and propyl gallate. Examples of suitable hydrophobic materials include dibutyl adipate, diisopropyl adipate, other adipic acid esters, petrolatum, mineral oil, silicone oil, cocoa butter, hexyl laurate, isopropyl myristate, propylene glycol dipelargonate, glyceryl stearate, beeswax, white wax, hydrocarbon wax, sperm wax, lanolin and cetyl palmitate. The formulation may also contain from about 1 to about 25% by weight of ammonium lactate, a topically active medicament used for relief of dry skin and hyperkeratotic conditions such as ichthyosis.

The formulations of this invention may be used in the treatment of acne or other dermatological conditions where tretinoin is indicated or to retard the effects of aging of the skin. The formulations are applied topically to the area to be treated at regular intervals, as needed. The duration of the treatment will depend upon the nature and severity of the condition to be treated as well as the frequency of application of the formulation.

There are provided the following eight tretinoin cream formulations which are stable both physically and chemically for long periods of time, and which do not contain xanthan gum:

| Ingredient | Parts by Weight | | Function |
| | Example 1 | Example 2 | |
| --- | --- | --- | --- |
| Tretinoin | 0.1 | 0.1 | Active Ingredient |
| Polyoxyethylene-2-stearyl ether | 2.5 | 2.5 | Nonionic emulsifier |
| Polyoxyethylene-21-stearyl ether | 2.5 | 2.5 | Nonionic emulsifier |
| Cetyl alcohol | 3.0 | 3.0 | Emollient/thickening agent |
| Stearyl alcohol | 6.0 | 5.0 | Emollient/thickening agent |
| Glycerin | 4.0 | 2.0 | Moisturizer |
| Dibutyl adipate | 20.0 | 20.0 | Solvent for tretinoin and emollient |
| Citric acid | 0.005 | 0.0039 | Chelating agent |
| Polyoxyethylene-4-lauryl ether | 1.00 | 1.00 | Solvent for tretinoin and nonionic emulsifier |
| Glyceryl stearate | 1.00 | 1.00 | Coemulsifier |
| Butylated hydroxytoluene | 0.05 | 0.05 | Antioxidant |
| Butylated hydroxyanisole | 0.05 | 0.05 | Antioxidant |
| Diazolidinyl urea | 0.15 | 0.15 | Preservative |
| Methylchloroisothiazolinone and methylisothiazolinone (Kathon CG$^{TM}$) | 0.05 | 0.05 | Preservative |
| Cocoa butter | – | 8.00 | Emollient |
| Hexyl laurate | – | – | Solvent for tretinoin and emollient |
| Methyl gluceth P-20 | – | – | Moisturizer |
| Propylene glycol dipelargonate | – | – | Solvent for tretinoin and emollient |
| Isopropyl Myristate | – | – | Solvent for tretinoin and emollient |
| Purified Water, USP | 59.60 | 54.59 | Vehicle |

| Ingredient | Parts by Weight | | Function |
|---|---|---|---|
| | Example 3 | Example 4 | |
| Tretinoin | 0.01 | 0.05 | Active Ingredient |
| Polyoxyethylene-2-stearyl ether | 2.00 | 2.5 | Nonionic emulsifier |
| Polyoxyethylene-21-stearyl ether | 2.00 | 2.5 | Nonionic emulsifier |
| Cetyl alcohol | 3.00 | 3.00 | Emollient/thickening agent |
| Stearyl alcohol | 6.00 | 5.00 | Emollient/thickening agent |
| Glycerin | 2.00 | 2.00 | Moisturizer |
| Dibutyl adipate | – | – | Solvent for tretinoin and emollient |
| Citric acid | 0.0039 | 0.0039 | Chelating agent |
| Polyoxyethylene-4-lauryl ether | 1.00 | 1.00 | Solvent for tretinoin and nonionic emulsifier |
| Glyceryl stearate | 1.00 | 1.00 | Coemulsifier |
| Butylated hydroxytoluene | 0.05 | 0.05 | Antioxidant |
| Butylated hydroxyanisole | 0.05 | 0.05 | Antioxidant |
| Diazolidinyl urea | 0.10 | 0.20 | Preservative |
| Methylchloroisothiazolinone and methylisothiazolinone (Kathon CG$^{TM}$) | 0.05 | 0.05 | Preservative |
| Cocoa butter | – | 8.00 | Emollient |
| Hexyl laurate | 10.00 | – | Solvent for tretinoin and emollient |
| Methyl gluceth P-20 | 2.00 | – | Moisturizer |
| Propylene glycol dipelargonate | – | 5.00 | Solvent for tretinoin and emollient |
| Isopropyl Myristate | – | 5.00 | Solvent for tretinoin and emollient |
| Purified Water, USP | 70.73 | 64.59 | Vehicle |

| Ingredient | Parts by Weight | | Function |
|---|---|---|---|
| | Example 5 | Example 6 | |
| Tretinoin | 0.1 | 0.1 | Active ingredient |
| Dibutyl adipate | 20.0 | 20.0 | Solvent for tretinoin |
| Cetyl alcohol | 3.0 | 3.0 | Emollient/thickening agent |
| Stearyl alcohol | 6.0 | 6.0 | Emollient/thickening agent |
| Glyceryl stearate | 1.0 | 1.0 | Coemulsifier |
| Butylated hydroxytoluene | 0.05 | 0.05 | Antioxidant |
| Butylated hydroxyanisole | 0.05 | 0.05 | Antioxidant |
| Sodium lauryl sulfate | 1.00 | --- | Anionic emulsifier |
| Cetyl trimethyl ammonium bromide | --- | 1.00 | Cationic emulsifier |
| Glycerin | 3.00 | 3.00 | Moisturizer |
| Diazolidinyl urea | 0.10 | 0.10 | Preservative |
| Kathon CG$^{TM}$ | 0.05 | 0.50 | Preservative |
| Disodium EDTA | 0.05 | --- | Chelating agent |
| Purified Water, USP | 65.6 | 65.6 | Vehicle |

EP 0 408 370 B1

| Ingredient | Parts by Weight | | Function |
|---|---|---|---|
| | Example 7 | Example 8 | |
| Tretinoin | 0.05 | 0.05 | Active ingredient |
| Mineral oil | --- | 10.00 | Emollient |
| Dibutyl adipate | 10.00 | --- | Solvent for tretinoin and emollient |
| Ammonium lactate | 21.35 | 21.35 | Active ingredient |
| Polyoxyethylene-2-stearyl ether | 2.5 | 2.5 | Nonionic emulsifier |
| Polyoxyethylene-21-stearyl ether | 2.5 | 2.5 | Nonionic emulsifier |
| Polyoxyethylene-4-lauryl ether | 1.0 | --- | Solvent for tretinoin and nonionic emulsifier |
| Cetyl alcohol | 3.0 | 3.0 | Emollient/thickening agent |
| Stearyl alcohol | 5.0 | 5.0 | Emollient/thickening agent |
| Butylated hydroxyanisole | 0.05 | 0.05 | Antioxidant |
| Butylated hydroxytoluene | 0.05 | 0.05 | Antioxidant |
| Glyceryl stearate | 1.00 | 1.00 | Coemulsifier |
| Glycerin | 4.00 | 4.00 | Moisturizer |
| Diazolidinyl urea | 0.10 | 0.10 | Preservative |
| Kathon CG™ | 0.05 | 0.05 | Preservative |
| Disodium EDTA | 0.05 | 0.05 | Chelating agent |
| Purified Water, USP | 49.299 | 50.299 | Vehicle |

The methods for processing Examples 1 to 4 are set forth below:

Example 1

1. In a suitable size premix vessel, warm the dibutyl adipate to 45-50 °C. With rapid mixing, add tretinoin and continue the mixing until uniform, while maintaining the temperature between 45-50 °C.

8

2. In a separate premix vessel, warm the following to 65°C: polyoxyethylene-2-stearyl ether, polyoxyethylene-21-stearyl ether, stearyl alcohol, butylated hydroxytoluene, butylated hydroxyanisole, glyceryl stearate, polyoxyethylene-4-lauryl ether and cetyl alcohol. Mix until uniform, maintaining the temperature between 60-65°C.

3. Add 95% of the water to the main mix vessel. While rapidly mixing, add the glycerin and citric acid. Mix until uniform and then warm and maintain the temperature at 60-65°C.

4. With rapid mixing, add the step 2 oil phase contents to step 3 water phase contents to form an emulsion. When the temperature of this emulsion is between 55-60°C, add step 1 tretinoin solution and continue rapid mixing while cooling to between 40-45°C. Adjust the pH of the cream in 3.8 to 4.5 range using citric acid.

5. In a small premix vessel, dissolve the diazolidinyl urea and Kathon CG™ in the remaining 5% of water. Add this solution to the step 4 cream and mix while cooling to room temperature.

Example 2

1. In a suitable size premix vessel, warm the dibutyl adipate to 45-50°C. With rapid mixing, add tretinoin and continue the mixing until uniform, while maintaining the temperature between 45-50°C.

2. In a separate premix vessel, warm the following to 65°C: cocoa butter, polyoxyethylene-2-stearyl ether, polyoxyethylene-21-stearyl ether, stearyl alcohol, butylated hydroxytoluene, butylated hydroxyanisole, glyceryl stearate, polyoxyethylene-4-lauryl ether and cetyl alcohol. Mix until uniform, maintaining the temperature between 60-65°C.

3. Add 95% of the water to the main mix vessel. While rapidly mixing, add the glycerin and citric acid. Mix until uniform and then warm and maintain the temperature at 60-65°C.

4. With rapid mixing, add the step 2 oil phase contents to step 3 water phase contents to form an emulsion. When the temperature of this emulsion is between 55-60°C, add step 1 tretinoin solution and continue rapid mixing while cooling to between 40-45°C. Adjust the pH of the cream in 3.8 to 4.5 range using citric acid.

5. In a small premix vessel, dissolve the diazolidinyl urea and Kathon CG™ in the remaining 5% of water. Add this solution to the step 4 cream and mix while cooling to room temperature.

Example 3

1. In a suitable size premix vessel, warm the hexyl laurate to 45-50°C. With rapid mixing, add tretinoin and continue the mixing until uniform, while maintaining the temperature between 45-50°C.

2. In a separate premix vessel, warm the following to 65°C: methyl gluceth P-20, polyoxyethylene-2-stearyl ether, polyoxyethylene-21-stearyl ether, stearyl alcohol, butylated hydroxytoluene, butylated hydroxyanisole, glyceryl stearate, polyoxyethylene-4-lauryl ether and cetyl alcohol. Mix until uniform, maintaining the temperature between 60-65°C.

3. Add 95% of the water to the main mix vessel. While rapidly mixing, add the glycerin and citric acid. Mix until uniform and then warm and maintain the temperature at 60-65°C.

4. With rapid mixing, add the step 2 oil phase contents to step 3 water phase contents to form an emulsion. When the temperature of this emulsion is between 55-60°C, add step 1 tretinoin solution and continue rapid mixing while cooling to between 40-45°C. Adjust the pH of the cream in 3.8 to 4.5 range using citric acid.

5. In a small premix vessel, dissolve the diazolidinyl urea and Kathon CG™ in the remaining 5% of water. Add this solution to the step 4 cream and mix while cooling to room temperature.

Example 4

1. In a suitable size premix vessel, warm the propylene glycol dipelargonate and isopropyl myristate to 45-50°C. With rapid mixing, add tretinoin and continue the mixing until uniform, while maintaining the temperature between 45-50°C.

2. In a separate premix vessel, warm the following to 65°C: cocoa butter, polyoxyethylene-2-stearyl ether, polyoxyethylene-21-stearyl ether, stearyl alcohol, butylated hydroxytoluene, butylated hydroxyanisole, glyceryl stearate, polyoxyethylene-4-lauryl ether and cetyl alcohol. Mix until uniform, maintaining the temperature between 60-65°C.

3. Add 95% of the water to the main mix vessel. While rapidly mixing, add the glycerin and citric acid. Mix until uniform and then warm and maintain the temperature at 60-65°C.

4. With rapid mixing, add the step 2 oil phase contents to step 3 water phase contents to form an emulsion. When the temperature of this emulsion is between 55-60°C, add step 1 tretinoin solution and continue rapid mixing while cooling to between 40-45°C. Adjust the pH of the cream in 3.8 to 4.5 range using citric acid.

5. In a small premix vessel, dissolve the diazolidinyl urea and Kathon CG™ in the remaining 5% of water. Add this solution to the step 4 cream and mix while cooling to room temperature.

Table 1 sets forth the concentrations of tretinoin in the cream formulations of Examples 1-4 at room temperature (RT) and at 40°C as determined at different time intervals. It will be appreciated that experimental error gave rise to those instances wherein more than 100% of tretinoin was found to be in the formulation after storage.

Hereinafter, "% (w/w)" means the percent by weight of tretinoin based upon the weight of the total composition; and "% Initial" means the percent of tretinoin remaining after storage for the designated times and temperatures based upon the initial amount of tretinoin in the formulation. Tretinoin content in these formulations was measured by stability indicating HPLC assay. Table 1 illustrates that the chemical stability of the emulsified tretinoin cream formulations of this invention is very high.

The physical stability of the emulsified tretinoin cream formulations of this invention is also very high. Thus, these formulations show no separation when stored at 40°C (104°F) for 26 weeks.

TABLE 1

| Time Temperature | Example 1 %(w/w) | % Initial | Example 2 %(w/w) | % Initial | Example 3 %(w/w) | % Initial | Example 4 %(w/w) | % Initial |
|---|---|---|---|---|---|---|---|---|
| Initial | 0.107 | | 0.103 | | 0.0137 | | 0.0518 | |
| 4 Week 40°C | 0.108 | (101) | 0.103 | (100) | 0.0135 | (98.54) | 0.053 | (102.3) |
| 8 Week 40°C | 0.108 | (101) | 0.104 | (101) | 0.0131 | (95.60) | 0.0524 | (101.1) |
| 12 Week 40°C | 0.106 | (99.06) | 0.100 | (97.08) | 0.0133 | (97.08) | 0.0522 | (100.7) |
| 26 Week 40°C | 0.102 | (95.3) | 0.098 | (95.1) | 0.0126 | (92.0) | -- | -- |
| 12 Week RT | 0.108 | (101) | 0.102 | (99.00) | 0.0139 | (101.0) | 0.0534 | (103.00) |
| 26 Week RT | 0.107 | (100) | 0.102 | (99.00) | 0.0136 | (99.3) | 0.0536 | (103.47) |
| 1 Year RT | 0.108 | (101) | 0.104 | (101.00) | 0.0132 | (96.4) | 0.0524 | (101.1) |

Table 2 sets forth the concentrations of tretinoin in the cream formulations of Examples 5-8 at room temperature (RT) and at 40°C as determined at different time intervals. Experimental error gave rise to those instances where more than 100% of tretinoin was found to be in the formulation after storage.

TABLE 2

| Time / Temperature | Example 5 | | Example 6 | | Example 7 | | Example 8 | |
|---|---|---|---|---|---|---|---|---|
| | % (w/w) | % Initial | % (w/w) | % Initial | % (w/w) | % Initial | % (w/w) | % Initial |
| Initial | 0.104 | | 0.0986 | | 0.0475 | | 0.05 | |
| 4 Week 40°C | 0.104 | (100) | 0.0972 | (98.5) | 0.0477 | (100.4) | 0.05 | (100) |
| 8 Week 40°C | 0.101 | (97.1) | 0.0948 | (95.5) | 0.0467 | (98.3) | Not evaluated | |
| 12 Week 40°C | 0.0989 | (95.0) | Not evaluated | | 0.0455 | (95.7) | 0.051 | (102) |
| 8 Week RT | 0.106 | (102) | 0.0972 | (98.5) | 0.0479 | (100.8) | 0.0510 | (100) |
| 12 Week RT | 0.104 | (100) | 0.102 | (103.4) | 0.049 | (103.1) | 0.0496 | (99.2) |

Examples 9 and 10 illustrate the necessity of using at least 4.0% by weight of stearyl alcohol or 15% by weight of cetyl alcohol and the synergism achieved by the use of stearyl alcohol in combination with cetyl alcohol.

Example 9

The basic formulation was as follows:

| Ingredient | % w/w |
|---|---|
| Tretinoin | 0.10 |
| Dibutyl adipate | 20.0 |
| Polyoxyethylene-21-stearyl ether | 2.5 |
| Polyoxyethylene-2-stearyl ether | 2.5 |
| Glyceryl stearate | 1.0 |
| Polyoxyethylene-4-lauryl ether | 1.0 |
| Cetyl alcohol | Test variable |
| Stearyl alcohol | Test variable |
| Glycerin | 4.0 |
| Kathon CG™ | 0.05 |
| Diazolidinyl urea | 0.10 |
| Butylated hydroxytoluene | 0.05 |
| Butylated hydroxyanisole | 0.05 |
| Citric acid | 0.005 |
| Water, purified | qs100.00 |

The following formulations were provided:

| Example No. | % w/w Stearyl Alcohol | % w/w Cetyl Alcohol |
|---|---|---|
| 9A | 6 | 3 |
| 9B | 0 | 3 |
| 9C | 2 | 3 |
| 9D | 3 | 3 |
| 9E | 4 | 3 |
| 9F | 4 | 0 |
| 9G | 6 | 0 |
| 9H | 9 | 0 |
| 9I | - | 9 |
| 9J | - | 15 |
| 9K | - | 20 |

The physical stabilities of these examples at 45°C (113°F), which are given in Table 3, illustrate the amounts of stearyl alcohol and/or cetyl alcohol required to stabilize tretinoin formulations.

13

TABLE 3

| Time | Example | | | | | | | | | | |
|------|------|------|------|------|------|------|------|------|------|------|------|
| | 9A | 9B | 9C | 9D | 9E | 9F | 9G | 9H | 9I | 9J | 9K |
| 1 week | N | S | S | N | N | N | N | N | N | N | N |
| 2 week | N | - | - | S | N | S | N | N | N | N | N |
| 3 week | N | - | - | - | N | - | N | N | S | N | N |
| 4 week | N | - | - | - | N | - | N | N | - | N | N |
| 5 week | N | - | - | - | N | - | N | N | - | N | N |
| 6 week | N | - | - | - | N | - | N | N | - | N | N |
| 7 week | N | - | - | - | N | - | N | N | - | N | N |
| 8 week | N | - | - | - | N | - | N | N | - | N | N |
| 9 week | N | - | - | - | N | - | N | N | - | N | N |
| 10 week | N | - | - | - | N | - | N | N | - | N | N |

In the above Table, "N" means no separation and "S" means that the oil and water phase is separated. As a rule, emulsions showing no separation when stored at 45°C (113°F) for eight weeks should be stable for a minimum of two years when stored at room temperature.

Example 10

The following formulations were prepared.

| Ingredient | % w/w of Ingredient in Example Number | | | | | |
|------------|------|------|------|------|------|------|
| | 10A | 10B | 10C | 10D | 10E | 10F |
| Tretinoin | 0.125 | 0.125 | 0.125 | 0.125 | 0.125 | 0.125 |
| Isopropyl myristate | 10.00 | 10.00 | 10.00 | 10.00 | 10.00 | 10.00 |
| Polyoxyl 40 stearate | 5.00 | 5.00 | 2.50 | 5.00 | 5.00 | 5.00 |
| Stearyl alcohol | 3.00 | 7.00 | 7.00 | 7.00 | 7.00 | 3.00 |
| Stearic acid | 19.00 | 19.00 | 19.00 | 19.00 | 12.00 | 19.00 |
| Butylated hydroxytoluene | 0.10 | 0.10 | 0.10 | 0.10 | 0.10 | 0.10 |
| Polyoxyethylene-2-stearyl ether | 0 | 1.45 | 2.50 | 0 | 0 | 1.66 |
| Cetyl alcohol | 0 | 3.00 | 3.00 | 3.00 | 3.00 | 0 |
| Sorbic acid | 0.20 | 0.20 | 0.20 | 0.20 | 0.20 | 0.2 |
| Water purified qs | 100.00 | 100.00 | 100.00 | 100.00 | 100.00 | 100.00 |

In formulations 10B and 10C, the HLB was adjusted using a blend of emulsifiers (polyoxyl-40-stearate and polyoxyethylene-2-stearyl ether), as well as including cetyl and stearyl alcohol. Thus, formulations 10B and 10C illustrate the importance of an HLB adjustment and the effects of cetyl alcohol and stearyl alcohol. Formulas 10D and 10E contain cetyl and stearyl alcohol, but provide no HLB adjustment, whereas formulation 10F had only HLB adjustments. The physical stabilities of these tretinoin creams at 45°C are shown in Table 4.

14

TABLE 4

| Time | Formulation Number | | | | | |
|------|-----|-----|-----|-----|-----|-----|
|      | 10A | 10B | 10C | 10D | 10E | 10F |
| 1 week | N | N | N | N | N | N |
| 2 week | S | N | N | N | N | S |
| 3 week | - | N | N | N | N | - |
| 4 week | - | N | N | N | N | - |
| 5 week | - | N | N | N | N | - |
| 6 week | - | N | N | N | N | - |
| 7 week | - | N | N | N | N | - |
| 8 week | - | N | N | N | N | - |
| 9 week | - | N | N | N | N | - |
| 10 week | - | N | N | S | N | - |

In Table 4, the designations "N" and "S" have the same meanings as discussed previously with respect to Table 3. Formulations 10A and 10F have reduced stabilizing thickener (stearyl alcohol). Neither formulation is physically stable. HLB adjustment in formulation 10F was not adequate to stabilize the formulation 10A. Formulations 10B, 10C, 10D and 10E demonstrate that with adequate amounts of stearyl alcohol and cetyl alcohol, the formulation 10A can be stabilized with or without HLB adjustment.

**Claims**

**Claims for the following Contracting States : AT, BE, CH, DE, DK, FR, GB, IT, LU, NL, SE**

1. The use of either
   (a) from 4 to 30% by weight of stearyl alcohol, or
   (b) 12 to 30% by weight of cetyl alcohol, or
   (c) cetyl alcohol and 4 to 30% by weight of stearyl alcohol to stabilise an emulsified aqueous tretinoin cream formulation comprising a therapeutically active amount of tretinoin and a pharmaceutically acceptable aqueous carrier, whereby breaking of the emulsion is prevented.

2. The use of claim 1 wherein the formulation comprises from 0.005 to 0.5% by weight of tretinoin; from 1 to 15% by weight of primary emulsifier; from 10 to 50% by weight of hydrophobic material; from 0.05 to 5% by weight of preservatives; from 0.01 to 1% by weight of antioxidants; and water.

3. The use of claim 2 wherein said hydrophobic material is selected from fatty acids having 12 to 20 carbon atoms, fatty acid esters in which the fatty acid portion has 6 to 20 carbon atoms, fatty alcohols having 12 to 20 carbon atoms, cocoa butter, mineral oil, petroleum, beeswax, white wax, hydrocarbon wax, sperm wax, and silicone oils.

4. The use of claim 2 or claim 3 wherein the primary emulsifier is a non-ionic emulsifier.

5. The use of claim 2 or claim 3 wherein the primary emulsifier is non-ionic, cationic, anionic or a mixture thereof.

6. The use of claim 4 wherein the emulsifier is selected from polyoxyethylene-2-stearyl ether, polyoxyethylene-21-stearyl ether, polyoxy-ethylene-4-lauryl ether, polyethylene glycol-23-lauryl ether, sorbitan monostearate and polyoxythlene-20-sorbitan monostearate.

7. The use of claim 5 wherein the anionic emulsifier is sodium lauryl sulfate and/or the cationic emulsifier is cetyl trimethyl ammonium bromide.

8. The use of any one of claims 1 to 7 wherein the formulation contains a preservative selected from diazolidinyl urea, benzyl alcohol, methyl paraben, propyl paraben, DMDM hydantoin, iodo propynyl butyl carbamate and methylisothiazolinone, and methylchloroisothiazolinone.

9. The use of any one of claims 1 to 8 wherein the formulation contains an antioxidant selected from butylated hydroxyanisole, butylated hydroxytoluene, tocopherol and propyl gallate.

10. The use of any one of claims 1 to 9 wherein the formulation contains from about 1 to about 25% by weight of ammonium lactate.

11. The use of any one of claims 1 to 10 wherein the stabilizer comprises a combination of from 4 to 30% by weight of stearyl alcohol and from 3 to 6% by weight of cetyl alcohol.

12. The use according to any one of claims 1 to 10 wherein the stabilizer comprises either 6-9% by weight of stearyl alcohol or a combination of 4-30% by weight of stearyl alcohol with at least 3% by weight cetyl alcohol.

13. A stable emulsified tretinoin cream formulation which does not contain xanthan gum, containing 0.1 parts by weight tretinoin, 2.5 parts by weight polyoxyethylene-2-stearyl ether, 2.5 parts by weight polyoxyethylene-21-stearyl ether, 3.0 parts by weight cetyl alcohol, 6.0 parts by weight stearyl alcohol, 4.0 parts by weight glycerin, 20.0 parts by weight dibutyl adipate, 0.005 parts by weight citric acid, 1.00 parts by weight polyoxyethylene-4-lauryl ether, 1.00 parts by weight glyceryl stearate, 0.05 parts by weight butylated hydroxytoluene, 0.05 parts by weight butylated hydroxyanisole, 0.15 parts by weight diazolidinyl urea, a mixture of 0.05 percent methylchloroisothiazolinone and methylisothiazolinone, and 59.60 parts by weight purified water.

14. A stable emulsified tretinoin cream formulation which does not contain xanthan gum, containing 0.1 parts by weight tretinoin, 2.5 parts by weight polyoxyethylene-2-stearyl ether, 2.5 parts by weight polyoxyethylene-21-stearyl ether, 3.0 parts by weight cetyl alcohol, 5.0 parts by weight stearyl alcohol, 2.0 parts by weight glycerin, 20.0 parts by weight dibutyl adipate, 0.0039 parts by weight citric acid, 1.00 parts by weight polyoxyethylene-4-lauryl ether, 1.00 parts by weight glyceryl stearate, 0.05 parts by weight butylated hydroxytoluene, 0.05 parts by weight butylated hydroxyanisole, 0.15 parts by weight diazolidinyl urea, a mixture of 0.05 parts by weight methylchloroisothiazolinone and methylisothiazolinone, 8.00 parts by weight cocoa butter, and 54.59 parts by weight purified water.

15. A stable emulsified tretinoin cream formulation which does not contain xanthan gum, containing 0.01 parts by weight tretinoin, 2.00 parts by weight polyoxyethylene-2-stearyl ether, 2.00 parts by weight polyoxyethylene-21-stearyl ether, 3.00 parts by weight cetyl alcohol, 6.00 parts by weight stearyl alcohol, 2.00 parts by weight glycerin, 0.0039 parts by weight citric acid, 1.00 parts by weight polyoxyethylene-4-lauryl ether, 1.00 parts by weight glyceryl stearate, 0.05 parts by weight butylated hydroxytoluene, 0.05 parts by weight butylated hydroxyanisole, 0.10 parts by weight diazolidinyl urea, a mixture of 0.05 parts by weight methylchloroisothiazolinone and methylisothiazolinone, 10.00 parts by weight hexyl laurate, 2.00 parts by weight methyl gluceth P-20, and 70.73 parts by weight purified water.

16. A stable emulsified tretinoin cream formulation which does not contain xanthan gum containing 0.05 parts by weight tretinoin, 2.5 parts by weight polyoxyethylene-2-stearyl ether, 2.5 parts by weight polyoxyethylene-21-stearyl ether, 3.00 parts by weight cetyl alcohol, 5.00 parts by weight stearyl alcohol, 2.00 parts by weight glycerin, 0.0039 parts by weight citric acid, 1.00 parts by weight polyoxyethylene-4-lauryl ether, 1.00 parts by weight glyceryl stearate, 0.05 parts by weight butylated hydroxytoluene, 0.05 parts by weight butylated hydroxyanisole, 0.20 parts by weight diazolidinyl urea, a mixture of 0.05 parts by weight methylchloroisothiazolinone and methylisothiazolinone, 8.00 parts by weight cocoa butter, 5.00 parts by weight propylene glycol dipelargonate, 5.00 parts by weight isopropyl myristate, and 64.59 parts by weight purified water.

17. Formulation as claimed in any one of claims 13 to 15 for use in the treatment of dermatological conditions.

18. The formulation of claim 17 wherein the dermatological condition is *acne vulgaris*.

19. A method for producing a stable emulsified aqueous tretinoin cream formulation wherein the ingredients specified in any one of the preceding claims are emulsified.

16

**Claims for the following Contracting States : ES, GR**

1. A process for stabilising an emulsified aqueous tretinoin cream formulation comprising a therapeutically active amount of tretinoin and a pharmaceutically acceptable aqueous carrier; which process comprises emulsifying said tretinoin and carrier with either
   (a) from 4 to 30% by weight of stearyl alcohol, or
   (b) 12 to 30% by weight of cetyl alcohol, or
   (c) cetyl alcohol and 4 to 30% by weight of stearyl alcohol as the stabiliser, whereby breaking of the emulsion is prevented.

2. The process of claim 1 wherein the formulation contains from 0.005 to 0.5% by weight of tretinoin; from 1 to 15% by weight of primary emulsifier; from 10 to 50% by weight of hydrophobic material; from 0.05 to 5% by weight of preservatives; from 0.01 to 1% by weight of antioxidants; and water.

3. The process of claim 2 wherein said hydrophobic material is selected from fatty acids having 12 to 20 carbon atoms, fatty acid esters in which the fatty acid portion has 6 to 20 carbon atoms, fatty alcohols having 12 to 20 carbon atoms, cocoa butter, mineral oil, petroleum, beeswax, white wax, hydrocarbon wax, sperm wax, and silicone oils.

4. The process of claim 2 or claim 3 wherein the primary emulsifier is a non-ionic emulsifier.

5. The process of claim 2 or claim 3 wherein the primary emulsifier is non-ionic, cationic, anionic or a mixture thereof.

6. The process of claim 4 wherein the emulsifier is selected from polyoxyethylene-2-stearyl ether, polyoxyethylene-21-stearyl ether, polyoxy-ethylene-4-lauryl ether, polyethylene glycol-23-lauryl ether, sorbitan monostearate and polyoxyethylene-20-sorbitan monostearate.

7. The process of claim 5 wherein the anionic emulsifier is sodium lauryl sulphate and/or the cationic emulsifier is cetyl trimethyl ammonium bromide.

8. The process of any one of claims 1 to 7 wherein the formulation contains a preservative selected from diazolidinyl urea, benzyl alcohol, methyl paraben, propyl paraben, DMDM hydantoin, iodo propynyl butyl carbamate and methylisothiazolinone, and methylchloroisothiazolinone.

9. The process of any one of claims 1 to 8 wherein the formulation contains an antioxidant selected from butylated hydroxyanisole, butylated hydroxytoluene, tocopherol and propyl gallate.

10. The process of any one of claims 1 to 9 wherein the formulation contains from 1 to 25% by weight of ammonium lactate.

11. The process of any one of claims 1 to 10 wherein the stabiliser comprises a combination of from 4 to 30% by weight of stearyl alcohol and from 3 to 6% by weight of cetyl alcohol.

12. The process of any of claims 1 to 10 wherein the stabiliser comprises either 6-9% by weight of stearyl alcohol or a combination of 4-30% by weight of stearyl alcohol with at least 3% by weight cetyl alcohol.

13. A process for making a stable emulsified tretinoin cream formulation which does not contain xanthan gum, comprising emulsifying 0.1 parts by weight tretinoin, 2.5 parts by weight polyoxyethylene-2-stearyl ether, 2.5 parts by weight polyoxyethylene-21-stearyl ether, 3.0 parts by weight cetyl alcohol, 6.0 parts by weight stearyl alcohol, 4.0 parts by weight glycerin, 20.0 parts by weight dibutyl adipate, 0.005 parts by weight citric acid, 1.00 parts by weight polyoxyethylene-4-lauryl ether, 1.00 parts by weight glyceryl stearate, 0.05 parts by weight butylated hydroxytoluene, 0.05 parts by weight butylated hydroxyanisole, 0.15 parts by weight diazolidinyl urea, a mixture of 0.05 percent methylchloroisothiazolinone and methylisothiazolinone, and 59.60 parts by weight purified water.

14. A process for making a stable emulsified tretinoin cream formulation which does not contain xanthan gum, comprising emulsifying 0.1 parts by weight tretinoin, 2.5 parts by weight polyoxyethylene-2-stearyl ether, 2.5 parts by weight polyoxyethylene-2-stearyl ether, 3.0 parts by weight cetyl alcohol, 5.0 parts by weight stearyl alcohol, 2.0 parts by weight glycerin, 20.0 parts by weight dibutyl adipate, 0.0039 parts by weight citric acid, 1.00 parts by weight polyoxyethylene-4-lauryl ether, 1.00 parts by weight glyceryl stearate, 0.05 parts by weight butylated hydroxytoluene, 0.05 parts by weight butylated hydroxyanisole, 0.15 parts by weight diazolidinyl urea, a mixture of 0.05 parts by weight methylchoroisothiazolinone and methylisothiazolinone, 8.00 parts by weight cocoa butter, and 54.59 parts by weight purified water.

15. A process for making a stable emulsified tretinoin cream formulation which does not contain xanthan gum, comprising emulsifying 0.01 parts by weight tretinoin, 2.00 parts by weight polyoxyethylene-2-stearyl ether, 2.00 parts by weight polyoxyethylene-21-stearyl ether, 3.00 parts by weight cetyl alcohol, 6.00 parts by weight stearyl alcohol, 2.00 parts by weight glycerin, 0.0039 parts by weight citric acid, 1.00 parts by weight polyoxyethylene-4-lauryl ether, 1.00 parts by weight glyceryl stearate, 0.05 parts by weight butylated hydroxytoluene, 0.05 parts by weight butylated hydroxyanisole, 0.10 parts by weight diazolidinyl urea, a mixture of 0.05 parts by weight methylchloroisothiazolinone and methylisothiazolinone, 10.00 parts by weight hexyl laurate, 2.00 parts by weight methyl gluceth P-20, and 70.73 parts by weight purified water.

16. A process for making a stable emulsified tretinoin cream formulation which does not contain xanthan gum which comprises emulsifying together 0.05 parts by weight tretinoin, 2.5 parts by weight polyoxyethylene-2-stearyl ether, 2.5 parts by weight polyoxyethylene-21-stearyl ether, 3.00 parts by weight cetyl alcohol, 5.00 parts by weight stearyl alcohol, 2.00 parts by weight glycerin, 0.0039 parts by weight citric acid, 1.00 parts by weight polyoxyethylene-4-lauryl ether, 1.00 parts by weight glyceryl stearate, 0.05 parts by weight butylated hydroxytoluene, 0.05 parts by weight butylated hydroxyanisole, 0.20 parts by weight diazolidinyl urea, a mixture of 0.05 parts by weight methylchlorisothiazolinone and methylisothiazolinone, 8.00 parts by weight cocoa butter, 5.00 parts by weight propylene glycol dipelargonate, 5.00 parts by weight isopropyl myristate, and 64.59 parts by weight purified water.

**Patentansprüche**

**Patentansprüche für folgende Vertragsstaaten : AT, BE, CH, DE, DK, FR, GB, IT, LU, NL, SE**

1. Verwendung von
   (a) 4 bis 30 Gew.-% Stearylalkohol, oder
   (b) 12 bis 30 Gew.-% Cetylalkohol, oder
   (c) Cetylalkohol und 4 bis 30 Gew.-% Stearylalkohol, zur Stabilisierung einer emulgierten wäßrigen Tretinoincremeformulierung, die eine therapeutisch wirksame Menge an Tretinoin und einen pharmazeutisch akzeptablen wäßrigen Träger umfaßt, wobei ein Brechen der Emulsion verhindert wird.

2. Verwendung nach Anspruch 1, wobei die Formulierung 0,005 bis 0,5 Gew.-% Tretinoin; 1 bis 15 Gew.-% eines Primäremulgators; 10 bis 50 Gew.-% eines hydrophoben Materials; 0,05 bis 5 Gew.-% Konservierungsmittel; 0,01 bis 1 Gew.-% Antioxidanzien; und Wasser umfaßt.

3. Verwendung nach Anspruch 2, wobei das hydrophobe Material ausgewählt ist unter Fettsäuren mit 12 bis 20 Kohlenstoffatomen, Fettsäureestern, bei denen der Fettsäureteil 6 bis 20 Kohlenstoffatome aufweist, Fettalkoholen mit 12 bis 20 Kohlenstoffatomen, Kakaobutter, Mineralöl, Petroleum, Bienenwachs, weißes Wachs, Kohlenwasserstoffwachs, Spermacetwachs und Silikonölen.

4. Verwendung nach Anspruch 2 oder Anspruch 3, wobei der Primäremulgator ein nicht-ionischer Emulgator ist.

5. Verwendung nach Anspruch 2 oder Anspruch 3, wobei der Primäremulgator ein nicht-ionischer, kationischer, anionischer Emulgator oder ein Gemisch davon ist.

6. Verwendung nach Anspruch 4, wobei der Emulgator ausgewählt ist unter Polyoxyethylen-2-stearylether, Polyoxyethylen-21-stearylether, Polyoxyethylen-4-laurylether, Polyethylenglycol-23-laurylether, Sorbitanmonostearat und Polyoxyethylen-20-sorbitanmonostearat.

7. Verwendung nach Anspruch 5, wobei der anionische Emulgator Natriumlaurylsulfat ist und/oder der kationische Emulgator Cetyltrimethylammoniumbromid ist.

8. Verwendung nach einem der Ansprüche 1 bis 7, wobei die Formulierung ein Konservierungsmittel enthält, das ausgewählt ist unter Diazolidinylharnstoff, Benzylalkohol, Methylparaben, Propylparaben, DMDM-Hydantoin, Jodpropinyl-butylcarbamat und Methylisothiazolinon und Methylchlorisothiazolinon.

9. Verwendung nach einem der Ansprüche 1 bis 8, wobei die Formulierung ein Antioxidans enthält, das ausgewählt ist unter butyliertem Hydroxyanisol, butyliertem Hydroxytoluol, Tocopherol und Propylgallat.

10. Verwendung nach einem der Ansprüche 1 bis 9, wobei die Formulierung etwa 1 bis etwa 25 Gew.-% Ammoniumlactat enthält.

11. Verwendung nach einem der Ansprüche 1 bis 10, wobei der Stabilisator eine Kombination aus 4 bis 30 Gew.-% Stearylalkohol und 3 bis 6 Gew.-% Cetylalkohol umfaßt.

12. Verwendung nach einem der Ansprüche 1 bis 10, wobei der Stabilisator entweder 6 bis 9 Gew.-% Stearylalkohol oder eine Kombination aus 4 bis 30 Gew.-% Stearylalkohol mit wenigstens 3 Gew.-% Cetylalkohol umfaßt.

13. Stabile, emulgierte Tretinoincremeformulierung, die keinen Xanthangummi enthält, und die 0,1 Gewichtsteile Tretinoin, 2,5 Gewichtsteile Polyoxyethylen-2-stearylether, 2,5 Gewichtsteile Polyoxyethylen-21-stearylether, 3,0 Gewichtsteile Cetylalkohol, 6,0 Gewichtsteile Stearylalkohol, 4,0 Gewichtsteile Glycerin, 20,0 Gewichtsteile Dibutyladipat, 0,005 Gewichtsteile Zitronensäure, 1,00 Gewichtsteile Polyoxyethylen-4-laurylether, 1,00 Gewichtsteile Glycerylstearat, 0,05 Gewichtsteile butyliertes Hydroxytoluol, 0,05 Gewichtsteile butyliertes Hydroxyanisol, 0,15 Gewichtsteile Diazolidinylharnstoff, ein Gemisch aus 0,05 % Methylchlorisothiazolinon und Methylisothiazolinon und 59,60 Gewichtsteile gereinigtes Wasser enthält.

14. Stabile emulgierte Tretinoincremeformulierung, die keinen Xanthangummi enthält und die 0,1 Gewichtsteile Tretinoin, 2,5 Gewichtsteile Polyoxyethylen-2-stearylether, 2,5 Gewichtsteile Polyoxyethylen-21-stearylether, 3,0 Gewichtsteile Cetylalkohol, 5,0 Gewichtsteile Stearylalkohol, 2,0 Gewichtsteile Glycerin, 20,0 Gewichtsteile Dibutyladipat, 0,0039 Gewichtsteile Zitronensäure, 1,00 Gewichtsteile Polyoxyethylen-4-laurylether, 1,00 Gewichtsteile Glycerylstearat, 0,05 Gewichtsteile butyliertes Hydroxytoluol, 0,05 Gewichtsteile butyliertes Hydroxyanisol, 0,15 Gewichtsteile Diazolidinylharnstoff, ein Gemisch aus 0,05 Gewichtsteilen Methylchlorisothiazolinon und Methylisothiazolinon, 8,00 Gewichtsteile Kakaobutter und 54,59 Gewichtsteile gereinigtes Wasser enthält.

15. Stabile emulgierte Tretinoincremeformulierung, die keinen Xanthangummi enthält und die 0,01 Gewichtsteile Tretinoin, 2,00 Gewichtsteile Polyoxyethylen-2-stearylether, 2,00 Gewichtsteile Polyoxyethylen-21-stearylether, 3,00 Gewichtsteile Cetylalkohol, 6,00 Gewichtsteile Stearylalkohol, 2,00 Gewichtsteile Glycerin, 0,0039 Gewichtsteile Zitronensäure, 1,00 Gewichtsteile Polyoxyethylen-4-laurylether, 1,00 Gewichtsteile Glycerylstearat, 0,05 Gewichtsteile butyliertes Hydroxytoluol, 0,05 Gewichtsteile butyliertes Hydroxyanisol, 0,10 Gewichtsteile Diazolidinylharnstoff, ein Gemisch aus 0,05 Gewichtsteilen Methylchlorisothiazolinon und Methylisothiazolinon, 10,00 Gewichtsteile Hexyllaurat, 2,00 Gewichtsteile Methylgluceth P-20 und 70,73 Gewichtsteile gereinigtes Wasser enthält.

16. Stabile emulgierte Tretinoincremeformulierung, die keinen Xanthangummi enthält und die 0,05 Gewichtsteile Tretinoin, 2,5 Gewichtsteile Polyoxyethylen-2-stearylether, 2,5 Gewichtsteile Polyoxyethylen-21-stearylether, 3,00 Gewichtsteile Cetylalkohol, 5,00 Gewichtsteile Stearylalkohol, 2,00 Gewichtsteile Glycerin, 0,0039 Gewichtsteile Zitronensäure, 1,00 Gewichtsteile Polyoxyethylen-4-laurylether, 1,00 Gewichtsteile Glycerylstearat, 0,05 Gewichtsteile butyliertes Hydroxytoluol, 0,05 Gewichtsteile butyliertes Hydroxyanisol, 0,20 Gewichtsteile Diazolidinylharnstoff, ein Gemisch aus 0,05 Gewichtsteilen Methylchlorisothiazolinon und Methylisothiazolinon, 8,00 Gewichtsteile Kakaobutter, 5,00 Gewichtsteile Propylenglycoldipelargonat, 5,00 Gewichtsteile Isopropylmyristat und 64,59 Gewichtsteile gereinigtes Wasser enthält.

**17.** Formulierung nach einem der Ansprüche 13 bis 15 zur Anwendung bei der Behandlung dermatologischer Zustände.

**18.** Formulierung nach Anspruch 17, wobei es sich bei dem dermatologischen Zustand um Akne vulgaris handelt.

**19.** Verfahren zur Herstellung einer stabilen emulgierten wäßrigen Tretinoincremeformulierung, wobei die in einem der vorhergehenden Ansprüche spezifizierten Bestandteile emulgiert werden.

**Patentansprüche für folgende Vertragsstaaten : ES, GR**

**1.** Verfahren zur Herstellung einer emulgierten, wäßrigen Tretinoincremeformulierung, die eine therapeutisch wirksame Menge an Tretinoin und einen pharmazeutisch akzeptablen wäßrigen Träger umfaßt, wobei man das Tretinoin und den Träger mit
(a) 4 bis 30 Gew.-% Stearylalkohol, oder
(b) 12 bis 30 Gew.-% Cetylalkohol, oder
(c) Cetylalkohol und 4 bis 30 Gew.-% Stearylalkohol, emulgiert, wobei ein Brechen der Emulsion verhindert wird.

**2.** Verfahren nach Anspruch 1, wobei die Formulierung 0,005 bis 0,5 Gew.-% Tretinoin; 1 bis 15 Gew.-% eines Primäremulgators; 10 bis 50 Gew.-% eines hydrophoben Materials; 0,05 bis 5 Gew.-% Konservierungsmittel; 0,01 bis 1 Gew.-% Antioxidanzien; und Wasser umfaßt.

**3.** Verfahren nach Anspruch 2, wobei das hydrophobe Material ausgewählt ist unter Fettsäuren mit 12 bis 20 Kohlenstoffatomen, Fettsäureestern, bei denen der Fettsäureteil 6 bis 20 Kohlenstoffatome aufweist, Fettalkoholen mit 12 bis 20 Kohlenstoffatomen, Kakaobutter, Mineralöl, Petroleum, Bienenwachs, weißes Wachs, Kohlenwasserstoffwachs, Spermacetwachs und Silikonölen.

**4.** Verfahren nach Anspruch 2 oder Anspruch 3, wobei der Primäremulgator ein nicht-ionischer Emulgator ist.

**5.** Verfahren nach Anspruch 2 oder Anspruch 3, wobei der Primäremulgator ein nicht-ionischer, kationischer, anionischer Emulgator oder ein Gemisch davon ist.

**6.** Verfahren nach Anspruch 4, wobei der Emulgator ausgewählt ist unter Polyoxyethylen-2-stearylether, Polyoxyethylen-21-stearylether, Polyoxyethylen-4-laurylether, Polyethylenglycol-23-laurylether, Sorbitanmonostearat und Polyoxyethylen-20-sorbitanmonostearat.

**7.** Verfahren nach Anspruch 5, wobei der anionische Emulgator Natriumlaurylsulfat ist und/oder der kationische Emulgator Cetyltrimethylammoniumbromid ist.

**8.** Verfahren nach einem der Ansprüche 1 bis 7, wobei die Formulierung ein Konservierungsmittel enthält, das ausgewählt ist unter Diazolidinylharnstoff, Benzylalkohol, Methylparaben, Propylparaben, DMDM-Hydantoin, Jodpropinyl-butylcarbamat und Methylisothiazolinon und Methylchlorisothiazolinon.

**9.** Verfahren nach einem der Ansprüche 1 bis 8, wobei die Formulierung ein Antioxidans enthält, das ausgewählt ist unter butyliertem Hydroxyanisol, butyliertem Hydroxytoluol, Tocopherol und Propylgallat.

**10.** Verfahren nach einem der Ansprüche 1 bis 9, wobei die Formulierung etwa 1 bis etwa 25 Gew.-% Ammoniumlactat enthält.

**11.** Verfahren nach einem der Ansprüche 1 bis 10, wobei der Stabilisator eine Kombination aus 4 bis 30 Gew.-% Stearylalkohol und 3 bis 6 Gew.-% Cetylalkohol umfaßt.

**12.** Verfahren nach einem der Ansprüche 1 bis 10, wobei der Stabilisator entweder 6 bis 9 Gew.-% Stearylalkohol oder eine Kombination aus 4 bis 30 Gew.-% Stearylalkohol mit wenigstens 3 Gew.-% Cetylalkohol umfaßt.

**13.** Verfahren zur Herstellung einer stabilen, emulgierten Tretinoincremeformulierung, die keinen Xanthangummi enthält, wobei man 0,1 Gewichtsteile Tretinoin, 2,5 Gewichtsteile Polyoxyethylen-2-stearylether, 2,5 Gewichtsteile Polyoxyethylen-21-stearylether, 3,0 Gewichtsteile Cetylalkohol, 6,0 Gewichtsteile Stearylalkohol, 4,0 Gewichtsteile Glycerin, 20,0 Gewichtsteile Dibutyladipat, 0,005 Gewichtsteile Zitronensäure, 1,00 Gewichtsteile Polyoxyethylen-4-laurylether, 1,00 Gewichtsteile Glycerylstearat, 0,05 Gewichtsteile butyliertes Hydroxytoluol, 0,05 Gewichtsteile butyliertes Hydroxyanisol, 0,15 Gewichtsteile Diazolidinylharnstoff, ein Gemisch aus 0,05 % Methylchlorisothiazolinon und Methylisothiazolinon und 59,60 Gewichtsteile gereinigtes Wasser emulgiert.

**14.** Verfahren zur Herstellung einer stabilen emulgierten Tretinoincremeformulierung, die keinen Xanthangummi enthält, wobei man 0,1 Gewichtsteile Tretinoin, 2,5 Gewichtsteile Polyoxyethylen-2-stearylether, 2,5 Gewichtsteile Polyoxyethylen-21-stearylether, 3,0 Gewichtsteile Cetylalkohol, 5,0 Gewichtsteile Stearylalkohol, 2,0 Gewichtsteile Glycerin, 20,0 Gewichtsteile Dibutyladipat, 0,0039 Gewichtsteile Zitronensäure, 1,00 Gewichtsteile Polyoxyethylen-4-laurylether, 1,00 Gewichtsteile Glycerylstearat, 0,05 Gewichtsteile butyliertes Hydroxytoluol, 0,05 Gewichtsteile butyliertes Hydroxyanisol, 0,15 Gewichtsteile Diazolidinylharnstoff, ein Gemisch aus 0,05 Gewichtsteilen Methylchlorisothiazolinon und Methylisothiazolinon, 8,00 Gewichtsteile Kakaobutter und 54,59 Gewichtsteile gereinigtes Wasser emulgiert.

**15.** Verfahren zur Herstellung einer stabilen emulgierten Tretinoincremeformulierung, die keinen Xanthangummi enthält, wobei man 0,01 Gewichtsteile Tretinoin, 2,00 Gewichtsteile Polyoxyethylen-2-stearylether, 2,00 Gewichtsteile Polyoxyethylen-21-stearylether, 3,00 Gewichtsteile Cetylalkohol, 6,00 Gewichtsteile Stearylalkohol, 2,00 Gewichtsteile Glycerin, 0,0039 Gewichtsteile Zitronensäure, 1,00 Gewichtsteile Polyoxyethylen-4-laurylether, 1,00 Gewichtsteile Glycerylstearat, 0,05 Gewichtsteile butyliertes Hydroxytoluol, 0,05 Gewichtsteile butyliertes Hydroxyanisol, 0,10 Gewichtsteile Diazolidinylharnstoff, ein Gemisch aus 0,05 Gewichtsteilen Methylchlorisothiazolinon und Methylisothiazolinon, 10,00 Gewichtsteile Hexyllaurat, 2,00 Gewichtsteile Methylgluceth P-20 und 70,73 Gewichtsteile gereinigtes Wasser emulgiert.

**16.** Verfahren zur Herstellung einer stabilen emulgierten Tretinoincremeformulierung, die keinen Xanthangummi enthält, wobei man 0,05 Gewichtsteile Tretinoin, 2,5 Gewichtsteile Polyoxyethylen-2-stearylether, 2,5 Gewichtsteile Polyoxyethylen-21-stearylether, 3,00 Gewichtsteile Cetylalkohol, 5,00 Gewichtsteile Stearylalkohol, 2,00 Gewichtsteile Glycerin, 0,0039 Gewichtsteile Zitronensäure, 1,00 Gewichtsteile Polyoxyethylen-4-laurylether, 1,00 Gewichtsteile Glycerylstearat, 0,05 Gewichtsteile butyliertes Hydroxytoluol, 0,05 Gewichtsteile butyliertes Hydroxyanisol, 0,20 Gewichtsteile Diazolidinylharnstoff, ein Gemisch aus 0,05 Gewichtsteilen Methylchlorisothiazolinon und Methylisothiazolinon, 8,00 Gewichtsteile Kakaobutter, 5,00 Gewichtsteile Propylenglycoldipelargonat, 5,00 Gewichtsteile Isopropylmyristat und 64,59 Gewichtsteile gereinigtes Wasser emulgiert.

**Revendications**

**Revendications pour les Etats contractants suivants : AT, BE, CH, DE, DK, FR, GB, IT, LU, NL, SE**

**1.** Utilisation de soit:
(a) 4 à 30% en poids d'alcool stéarylique, soit
(b) 12 à 30% en poids d'alcool cétylique, soit
(c) de l'alcool cétylique et 4 à 30% en poids d'alcool stéarylique pour stabiliser une formule de crème de trétinoïne aqueuse émulsifiée comprenant une quantité thérapeutiquement active de trétinoïne et un support aqueux pharmaceutiquement acceptable, grâce à quoi la cassure de l'émulsion est empêchée.

**2.** Utilisation selon la revendication 1 dans laquelle la formule comprend 0,005 à 0,5% en poids de trétinoïne; 1 à 15% en poids d'émulsifiant primaire; 10 à 50% en poids de matériau hydrophobe; 0,05 à 5% en poids de conservateurs; 0,01 à 1% d'antioxydants; et de l'eau.

**3.** Utilisation selon la revendication 2, dans laquelle ledit matériau hydrophobe est sélectionné parmi des acides gras ayant 12 à 20 atomes de carbone, des esters d'acides gras dans lesquels la portion acide gras a 6 à 20 atomes de carbone, des alcools gras ayant 12 à 20 atomes de carbone, du beurre de cacao, de l'huile minérale, du pétrole, de la cire d'abeille, de la cire blanche, de la cire d'hydrocarbure, de la cire de spermacéti et des huiles de silicone.

4. Utilisation selon la revendication 2 ou la revendication 3 dans laquelle l'émulsifiant primaire est un émulsifiant non-ionique.

5. Utilisation selon la revendication 2 ou la revendication 3 dans laquelle l'émulsifiant primaire est non-ionique, cationique, anionique ou un de leurs mélanges.

6. Utilisation selon la revendication 4, dans laquelle l'émulsifiant est sélectionné parmi le polyoxyéthylène-2-stéaryléther, le polyoxyéthylène-21-stéaryléther, le polyoxy-éthylène-4-lauryléther, le polyéthylèneglycol-23-lauryléther, le monostéarate de sorbitanne et le monostéaarate de polyoxyéthylène-20-sorbitanne.

7. Utilisation selon la revendication 5, dans laquelle l'émulsifiant anionique est le lauryl sulfate de sodium et/ou l'émulsifiant cationique est le bromure de cétyl triméthyl ammonium.

8. Utilisation selon l'une quelconque des revendications 1 à 7 dans laquelle la formule contient un conservateur sélectionné parmi la diazolidinylurée, l'alcool benzylique, le méthylparabène, le propylparabène, la DMDM hydantoïne, l'iodo propynyl butyl carbamate et la méthylisothiazolinone, et la méthylchloroisothiazolinone.

9. Utilisation selon l'une quelconque des revendications 1 à 8 dans laquelle la formule contient un antioxydant sélectionné parmi l'hydroxyanisole butylé, l'hydroxytoluène butylé, le tocophérol et le gallate de propyle.

10. Utilisation selon l'une quelconque des revendications 1 à 9, dans laquelle la formule contient environ 1 à environ 25% en poids de lactate d'ammonium.

11. Utilisation selon l'une quelconque des revendications 1 à 10, dans laquelle le stabilisateur comprend une combinaison de 4 à 30% en poids d'alcool stéarylique et de 3 à 6% en poids d'alcool cétylique.

12. Utilisation selon l'une quelconque des revendications 1 à 10, dans laquelle le stabilisant comprend soit 6 à 9% en poids d'alcool stéarylique soit une combinaison de 4 à 30% en poids d'alcool stéarylique avec au moins 3% en poids d'alcool cétylique.

13. Formule de crème de trétinoïne émulsifiée stable qui ne contient pas de gomme xanthane, contenant 0,1 partie en poids de trétinoïne, 2,5 parties en poids de polyoxyéthylène-2-stéaryléther, 2,5 parties en poids de polyoxyéthylène-21-stéaryléther, 3,0 parties en poids d'alcool cétylique, 6,0 parties en poids d'alcool stéarylique, 4,0 parties en poids de glycérine, 20,0 parties en poids de dibutyladipate, 0,005 partie en poids d'acide citrique, 1,00 partie en poids de polyoxyéthylène-4-lauryléther, 1,00 partie en poids de stéarate de glycéryle, 0,05 partie en poids d'hydroxytoluène butylé, 0,05 partie en poids d'hydroxyanisole butylé, 0,15 partie en poids de diazolidinylurée, un mélange de 0,05 pourcent de méthylchloroisothiazolinone et de méthyliso-thiazolinone, et 59,60 parties en poids d'eau purifiée.

14. Formule de crème de trétinoïne émulsifiée stable qui ne contient pas de gomme xanthane, contenant 0,1 partie en poids de trétinoïne, 2,5 parties en poids de polyoxyéthylène-2-stéaryléther, 2,5 parties en poids de polyoxyéthylène-21-stéaryléther, 3,0 parties en poids d'alcool cétylique, 5,0 parties en poids d'alcool stéarylique, 2,0 parties en poids de glycérine, 20,0 parties en poids de dibutyladipate, 0,0039 partie en poids d'acide citrique, 1,00 partie en poids de polyoxyéthylène-4-lauryléther, 1,00 partie en poids de stéarate de glycéryle, 0,05 partie en poids d'hydroxytoluène butylé, 0,05 partie en poids d'hydroxyanisole butylé, 0,15 partie en poids de diazolidinylurée, un mélange de 0,05 partie en poids de méthylchloroisothiazolinone et de méthylisothiazolinone, 8,00 parties en poids de beurre de cacao, et 54,59 parties en poids d'eau purifiée.

15. Formule de crème de trétinoïne émulsifiée stable qui ne contient pas de gomme xanthane, contenant 0,01 partie en poids de trétinoïne, 2,00 parties en poids de polyoxyéthylène-2-stéaryléther, 2,00 parties en poids de polyoxyéthylène-21stéaryléther, 3,00 parties en poids d'alcool cétylique, 6,00 parties en poids d'alcool stéarylique, 2,00 parties en poids de glycérine, 0,0039 partie en poids d'acide citrique, 1,00 partie en poids de polyoxyéthylène-4-lauryléther, 1,00 partie en poids de stéarate de glycéryle, 0,05 partie en poids d'hydroxytoluène butylé, 0,05 partie en poids d'hydroxyanisole butylé, 0,10 partie en poids de diazolidinylurée, un mélange de 0,05 partie en poids de méthylchloroisothiazolinone et de méthylisothiazolinone, 10,00 parties en poids de laurate d'hexyle, 2,00 parties en poids de méthyl gluceth P-20, et 70,73 parties en poids d'eau purifiée.

16. Formule de crème de trétinoïne émulsifiée stable qui ne contient pas de gomme xanthane contenant 0,05 partie en poids de trétinoïne, 2,5 parties en poids de polyoxyéthylène-2-stéaryléther, 2,5 parties en poids de polyoxyéthylène-21-stéaryléther, 3,00 parties en poids d'alcool cétylique, 5,00 parties en poids d'alcool stéarylique, 2,00 parties en poids de glycérine, 0,0039 partie en poids d'acide citrique, 1,00 partie en poids de polyoxyéthylène-4-lauryléther, 1,00 partie en poids de stéarate de glycéryle, 0,05 partie en poids d'hydroxytoluène butylé, 0,05 partie en poids d'hydroxyanisole butylé, 0,20 partie en poids de diazolidinylurée, un mélange de 0,05 partie en poids de méthylchloroisothiazolinone et de méthylisothiazolinone, 8,00 parties en poids de beurre de cacao, 5,00 parties en poids de propylène glycol dipélargonate, 5,00 parties en poids de myristate d'isopropyle et 64,59 parties en poids d'eau purifiée.

17. Formule comme revendiquée dans l'une quelconque des revendications 13 à 15 pour utilisation dans le traitement de conditions dermatologiques.

18. Formule selon la revendication 17 dans laquelle la condition dermatologique est l'acné vulgaris.

19. Méthode pour produire une formule de crème de trétinoïne aqueuse émulsifiée stable dans laquelle les ingrédients spécifiés dans l'une quelconque des revendications précédentes sont émulsifiés.

**Revendications pour les Etats contractants suivants : ES, GR**

1. Procédé de stabilisation d'une formule de crème de trétinoïne aqueuse émulsifiée comprenant une quantité thérapeutiquement active de trétinoïne et un support aqueux pharmaceutiquement acceptable; lequel procédé comprend l'émulsification desdits trétinoïne et support avec soit:
(a) 4 à 30% en poids d'alcool stéarylique, soit
(b) 12 à 30% en poids d'alcool stéarylique, soit
(c) de l'alcool cétylique 4 à 30% en poids d'alcool stéarylique comme stabilisaant, grâce à quoi la cassure de l'émulsion est empêchée.

2. Procédé selon la revendication 1 dans lequel la formule comprend de 0,005 à 0,5% en poids de trétinoïne; 1 à 15% en poids d'émulsifiant primaire; 10 à 50% en poids de matériau hydrophobe; 0,05 à 5% en poids de conservateurs; 0,01 à 1% d'antioxydant; et de l'eau.

3. Procédé selon la revendication 2, dans lequel ledit matériau hydrophobe est sélectionné parmi des acides gras ayant 12 à 20 atomes de carbone, des esters d'acides gras dans lesquels la portion acide gras a 6 à 20 atomes de carbone, des alcools gras ayant 12 à 20 atomes de carbone, du beurre de cacao, de l'huile minérale, du pétrole, de la cire d'abeille, de la cire blanche, de la cire d'hydrocarbure, de la cire de spermacéti et des huiles de silicone.

4. Procédé selon la revendication 2 ou la revendication 3 dans lequel l'émulsifiant primaire est un émulsifiant non-ionique.

5. Procédé selon la revendication 2 ou la revendication 3 dans lequel l'émulsifiant primaire est non-ionique, cationique, anionique ou un de leurs mélanges.

6. Procédé selon la revendication 4, dans lequel l'émulsifiant est sélectionné parmi le polyoxyéthylène-2-stéaryléther, le polyoxyéthylène-21-stéaryléther, le polyoxyéthylène-4-lauryléther, le polyéthylèneglycol-23-lauryléther, le monostéarate de sorbitanne et le monostéarate de polyoxyéthylène-20-sorbitanne.

7. Procédé selon la revendication 5, dans lequel l'émulsifiant anionique est le lauryl sulfate de sodium et/ou l'émulsifiant cationique est le bromure de cétyl triméthyl ammonium.

8. Procédé selon l'une quelconque des revendications 1 à 7 dans lequel la formule contient un conservateur sélectionné parmi la diazolidinylurée, l'alcool benzylique, le méthylparabène, le propylparabène, la DMDM hydantoïne, l'iodo propynyl butyl carbamate et la méthylisothiazolinone, et la méthylchloroisothiazolinone.

9.  Procédé selon l'une quelconque des revendications 1 à 8 dans lequel la formule contient un antioxydant sélectionné parmi l'hydroxyanisole butylé, l'hydroxytoluène butylé, le tocophérol et le gallate de propyle.

10. Procédé selon l'une quelconque des revendications 1 à 9, dans lequel la formule contient 1 à 25% en poids de lactate d'ammonium.

11. Procédé selon l'une quelconque des revendications 1 à 10, dans lequel le stabilisant comprend une combinaison de 4 à 30% en poids d'alcool stéarylique et de 3 à 6% en poids d'alcool cétylique.

12. Procédé selon l'une quelconque des revendications 1 à 10, dans lequel le stabilisant comprend soit 6 à 9% en poids d'alcool stéarylique soit une combinaison de 4 à 30% en poids d'alcool stéarylique avec au moins 3% en poids d'alcool cétylique.

13. Procédé de fabrication d'une formule de crème de trétinoïne émulsifiés stable qui ne contient pas de gomme xanthane, comprenant l'émulsification de 0,1 partie en poids de trétinoïne, 2,5 parties en poids de polyoxyéthylène-2-stéaryléther, 2,5 parties en poids de polyoxyéthylène-21-stéaryléther, 3,0 parties en poids d'alcool cétylique, 6,0 parties en poids d'alcool stéarylique, 4,0 parties en poids de glycérine, 20,0 parties en poids de dibutyladipate, 0,005 partie en poids d'acide citrique, 1,00 partie en poids de polyoxyéthylène-4-lauryléther, 1,00 partie en poids de stéarate de glycéryle, 0,05 partie en poids d'hydroxytoluène butylé, 0,05 partie en poids d'hydroxyanisole butylé, 0,15 partie en poids de diazolidinylurée, un mélange de 0,05 % de méthylchloroisothiazolinone et de méthylisothiazolinone, et 59,60 parties en poids d'eau purifiée.

14. Procédé de fabrication d'une formule de crème de trétinoïne émulsifiée stable qui ne contient pas de gomme xanthane, comprenant l'émulsification de 0,1 partie en poids de trétinoïne, 2,5 parties en poids de polyoxyéthylène-2-stéaryléther, 2,5 parties en poids de polyoxyéthylène-21-stéaryléther, 3,0 parties en poids d'alcool cétylique, 5,0 parties en poids d'alcool stéarylique, 2,0 parties en poids de glycérine, 20,0 parties en poids de dibutyladipate, 0,0039 partie en poids d'acide citrique, 1,00 partie en poids de polyoxyéthylène-4-lauryléther, 1,00 partie en poids de stéarate de glycéryle, 0,05 partie en poids d'hydroxytoluène butylé, 0,05 partie en poids d'hydroxyanisole butylé, 0,15 partie en poids de diazolidinylurée, un mélange de 0,05 partie en poids de méthylchloroisothiazolinone et de méthyliso-thiazolinone, 8,00 parties en poids de beurre de cacao, et 54,59 parties en poids d'eau purifiée.

15. Procédé de fabrication d'une formule de crème de trétinoïne émulsifiée stable qui ne contient pas de gomme xanthane, comprenant l'émulsification de 0,01 partie en poids de trétinoïne, 2,00 parties en poids de polyoxyéthylène-2-stéaryléther, 2,00 parties en poids de polyoxyéthylène-21-stéaryléther, 3,00 parties en poids d'alcool cétylique, 6,00 parties en poids d'alcool stéarylique, 2,00 parties en poids de glycérine, 0,0039 partie en poids d'acide citrique, 1,00 partie en poids de polyoxyéthylène-4-lauryléther, 1,00 partie en poids de stéarate de glycéryle, 0,05 partie en poids d'hydroxytoluène butylé, 0,05 partie en poids d'hydroxyanisole butylé, 0,10 partie en poids de diazolidinylurée, un mélange de 0,05 partie en poids de méthylchloroisothiazolinone et de méthylisothiazolinone, 10,00 parties en poids de laurate d'hexyle, 2,00 parties en poids de méthyl gluceth P-20, et 70,73 parties en poids d'eau purifiée.

16. Procédé de fabrication d'une formule de crème de trétinoïne émulsifiée stable qui ne contient pas de gomme xanthane comprenant l'émulsification de 0,05 partie en poids de trétinoïne, 2,5 parties en poids de polyoxyéthylène-2-stéaryléther, 2,5 parties en poids de polyoxyéthylène-21-stéaryléther, 3,00 parties en poids d'alcool cétylique, 5,00 parties en poids d'alcool stéarylique, 2,00 parties en poids de glycérine, 0,0039 partie en poids d'acide citrique, 1,00 partie en poids de polyoxyéthylène-4-lauryléther, 1,00 partie en poids de stéarate de glycéryle, 0,05 partie en poids d'hydroxytoluène butylé, 0,05 partie en poids d'hydroxyanisole butylé, 0,20 partie en poids de diazolidinyl urée, un mélange de 0,05 parties en poids de méthylchloroisothiazolinone et de méthylisothiazolinone, 8,00 parties en poids de beurre de cacao, 5,00 parties en poids de dipélargonate de propylèneglycol, 5,00 parties en poids de myristate d'isopropyl et 64,59 parties en poids d'eau purifiée.